# EUROPEAN PATENT SPECIFICATION

(11) **EP 0 376 516 B1**
(45) Date of publication and mention of the grant of the patent: **10.02.1993**
(21) Application number: 89312781.1
(22) Date of filing: 07.12.1989
(51) Int. Cl.: C07C 49/255, C07C 45/51

(54) **Method of preparation of nabumetone**
Verfahren zur Herstellung von Nabumeton
Procédé de préparation de nabumétone

(30) Priority: 08.12.1988 US 282709
(43) Date of publication of application: 04.07.1990
(73) Proprietor: HOECHST CELANESE CORPORATION, Somerville, N.J. 08876 (US)
(72) Inventor: Aslam, Mohammad, Corpus Christi Texas (US); Davenport, Kenneth G., North Kingstown Rhode Island (US)
(74) Representative: De Minvielle-Devaux, Ian Benedict Peter

(56) References cited:
- FR-A- 2 314 912
- GB-A- 1 476 721
- NL-A- 8 803 088
- US-A- 3 922 299
- J. ORG. CHEM., vol. 41, no. 7, 1976, pages 1206-1209; A.J. CHALK et al.: "Palladium-catalyzed vinyl substitution reactions. II. Synthesis of aryl substituted allylic alcohols, aldehydes, and ketones from aryl halides and unsaturated alcohols"

## Description

This invention relates to a new method for the production of nabumetone, which may be also characterized as 4-(6′-methoxy-2′-naphthyl)-butan-2-one. Nabumetone is a well-known non-steroidal anti-inflamatory (NSAI) drug.

### Description of Related art

U. S. Patent No. 4,061,779, issued December 6, 1977, to W. L. Anthony et al, discloses the preparation of nabumetone by the hydrogenation of 4-(6′-methoxy-2′-naphthyl)-3-buten-2-one over a palladium on carbon catalyst (Example 21). The patent also teaches the preparation of nabumetone by reaction of 2-(6′-methoxy-2′-naphthyl)propionitrile with methyl magnesium iodide (Example 19) and reaction of 6-methoxy-2-bromomethylnaphthalene with acetylacetone (Example 22).

A. C. Goudie et al, Journal of Medicinal Chemistry, 1978, Vol. 21, No. 12, 1261-1264, also discloses the preparation of nabumetone by methods apparently identical with those of Examples 21 and 19 of U. S. Patent No. 4,061,779 discussed previously.

U. S. Patent No. 4,221,741, issued September 9, 1980, to L. M. Gaster, discloses the preparation of nabumetone by hydrogenation of 4-(6′-methoxy-2′-naphthyl)-4-hydroxy-but-3-en-2-one over a palladium on carbon catalyst.

A. J. Chalk et al, J. Org. Chem., Vol. 41, No. 2, 1976, 273-278, teach the synthesis of 2- and 3-phenyl substituted allylic alcohols, aldehydes and ketones by reaction of allylic alcohols with iodobenzene or bromobenzene in the presence of a palladium salt and a phosphine ligand. Included is the reaction of 3-butene-2-ol and bromobenzene resulting in a GC yield of 3-aryl carbonyl product of 70% with triphenyl phosphine and 80% with 1,2-bis(dimethylphosphino)ethane ligand.

A. J. Chalk et al, J. Org Chem., Vol, 41, No. 7, 1976, 1206-1209, is a continuation of the previously cited article and specifically discloses the reaction of 3-butene-2-ol with 4-acetylbromobenzene with palladium acetate and triphenyl phosphine as catalyst to produce 4-(4′-acetylphenyl)butane-2-ol in 35% yield isolated.

J. B. Melpolder et al, J. Org. Chem., Vol. 41, No. 2, 1976, 265-272, discloses palladium (II) catalyzed arylations of olefinic compounds with various aryl halides to produce arylated derivatives including the reaction of 3-buten-2-ol with bromobenzene and substituted bromobenzenes to produce phenylated and substituted phenylated butan-2-ones and the reaction of methyl acrylate with 1-bromonaphthalene to produce methyl-3-(1′-naphthyl)propenoate.

### SUMMARY OF THE INVENTION

In accordance with this invention, nabumetone is produced by a method comprising reacting 2-bromo-6-methoxynaphthalene with 3-buten-2-ol in the presence of a catalyst as shown in the following equation: 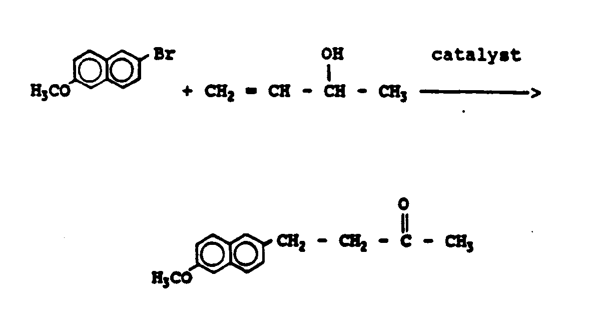
The method is capable of producing nabumetone in unpredicably high yields.

### DESCRIPTION OF PREFERRED EMBODIMENTS

The reaction is carried out at a temperature, for example, of from about 50°C to about 200°C, preferably about 75°C to about 175°C, and most preferably about 120°C to about 150°C, for a time sufficient to cause substantially complete reaction to occur. Typically the reaction time is from about 0.25 to about 10 hours, preferably from about 3 to about 6 hours.

The catalyst for the reaction is preferably a palladium compound complexed with at least one ligand comprising trivalent phosphorus. Some palladium catalyst which may be used wherein the palladium is complexed with an appropriate ligand are as follows: bis(triphenylphosphine)dichloro complex,
bis(tributylphosphine)dichloro complex,
bis(tricyclohexylphosphine)dichloro complex,
di-allyltriphenylphosphinedichloro complex,
triphenylphosphinepiperidinedichloro complex,
bis(cyclohexyloxime)dicarbonyl complex,
1,5,9-cyclododecatrienedichloro complex,
bis(triphenylphosphine)dicarbonyl complex,
bis(triphenylphosphine)diacetate complex,
bis(triphenylphosphine)sulfate complex,
2,4-pentanedionepalladium (II) complex,
tetrakis(triphenylphosphine) complex, and complexes in which some of the ligands are carbon monoxide such as chlorocarbonylbis(triphenylphosphine) complex, and the like, all complexes of palladium.

The palladium salts and phosphine ligands making up the foregoing catalyst complexes may also be added separately to the reaction zone. In this case, the amount of ligand added is preferably sufficient to complex with the palladium present such that the P:Pd mole ratio is equal to at least about 1:1 and may be as high as, for example, about 10:1. Preferably the P:Pd reatio is in the range of about 0.5:1 to 2:1. Salts of palladium (II) which may be utilized separately with the ligand are, for example, palladium (II) nitrate, chloride, acetate, bromide, sulfate and the like.

The reactants, 3-buten-2-ol and 2 bromo-6-methoxynaphthalene, may be mixed in equimolar quantities or one of the reactants may be used in excess. Preferably, an excess, typically of about 5 to 50% of the 3-buten-2-ol is used to drive the reaction to completion.

An equimolar quantity of the palladium catalyst may be used or, preferably, a lesser amount may be used in the presence of a compound which will regenerate the active catalyst during the reaction. Typically, from about 0.01 to about 0.1 molar amount of catalyst per mole of reactant is used.

Regeneration of the catalyst during reaction is accomplished by addition of an excess of base. As used in this description, a base is a compound which reacts with the hydrogen bromide formed in the reaction regenerating the catalyst and causing formation of an inert material. Ease compounds useful for the present invention include, for example, alkali metal bicarbonates and carbonates such as sodium bicarbonate, sodium carbonate, potassium bicarbonate, and potassium carbonate, and organic amines such as triethylamine, diethylamine, tripropylamine, and dipropylamine. The base may be used in an amount, for example, of about 1 to 5 mol per mole of 2-bromo-6-methoxynaphthalene. The preferred base is sodium bicarbonate which reacts with the hydrogen bromide to form carbon dioxide, water, and the active catalyst. The carbon dioxide and water are inert and are easily separable from the reaction solution on isolation of the product.

The reaction may be carried out without a solvent or in an inert liquid solvent. Solvents which can be used include, for example, nitriles, amides, and ethers, e.g., N-methyl pyrrolidone, acetonitrile, dimethyl sulfoxide, dimethyl formamide, dimethyl acetamide, tetrahydrofuran, and the like. The amount of solvent, if used, may be such that the weight ratio of solvent to total reaction mass, exclusive of the solvent, is up to about 5:1, preferably about 2:1.

The reaction is carried out in an inert atmosphere to avoid oxidation and by-product formation. Typically a nitrogen, argon, or helium atmosphere is used for the reaction.

The nabumetone product may be isolated from the reaction mixture by methods well known in the art. Typically, the reaction mixture may be poured into water at which time the product is separated as a crystalline solid. The solid may be recrystallized to give a pure crystalline material melting at about 80#C.

The following examples further illustrate the invention:

### Example 1

A mixture of 2-bromo-6-methoxynaphthalene (2.37 g, 10 mmol), 3-buten-2-ol (1.08 g, 15 mmol), palladium (II) acetate 0.02 g, 0.09 mmol), triphenylphosphine (0.047 9, 0.18 mmol), and sodium bicarbonate (1.0 g, 12 mmol) in N-methylpyrrolidone (10 mL) was heated at 140#C - 145#C under an inert atmosphere for 5 hours. At the end of the heating cycle, the reaction mixture was cooled to room temperature and poured in water to precipitate a solid. The solid was redissolved in dichloromethane (50 mL) and filtered through a celite pad to remove undissolved material. Concentration of the organic filtrate gave a solid (1.8 g). GC-analysis of the crude product suggested that the reaction went with complete conversion and contained 74% nabumetone.

### Example 2

A mixture of 2-bromo-6-methoxynaphthalene (2.37 g, 10 mmol), 3-buten-2-ol (1.08 g, 15 mmol), bis(triphenylphosphine) dichloro palladium (II) complex (0.13 9, 0.18 mmol), and sodium bicarbonate (1.0 g, 12 mmol) in N-methylpyrrolidone (10 mL) was heated at 140# under an inert atmosphere for 5 hours. At the end of the heating cycle, the reaction mixture was cooled to room temperature and poured in water (150 mL) to precipitate a solid. The solid was redissolved in dichloromethane (100 mL) and filtered through a celite pad to remove undissolved material. Concentration of the filtrate gave a solid (1.9 g). GC-analysis of the crude product suggested that the reaction went with complete conversion and contained 76% nabumetone.

## Claims

1. A method for the production of nabumetone which comprises reacting 2-bromo-6-methoxynaphthalene with 3-buten-2-ol in the presence of a catalyst.

2. The method of Claim 1 wherein the catalyst comprises a palladium compound in combination with a ligand comprising trivalent phosphorus.

3. The method of Claim 2 wherein the catalyst is palladium (II) acetate in combination with triphenyl phosphine.

4. The method of Claim 2 wherein the catalyst is a bis(triphenylphosphine) dichloropalladium (II) complex.

5. The method of any of Claims 1-4 wherein the reaction temperature is from 120°C to 150 °C.

6. The method of any of Claims 1-5 wherein the reaction time is from 0.25 to 10 hours.

7. The method of any of Claims 1-6 wherein the reaction is carried out in the presence of a solvent.

8. The method of Claim 7 wherein the solvent is N-methylpyrrolidone.

## Patentansprüche

1. Verfahren zur Herstellung von Nabumeton, umfassend die Reaktion von 2-Brom-6-methoxynaphthalin mit 3-Buten-2-ol in Anwesenheit eines Katalysators.

2. Verfahren nach Anspruch 1, worin der Katalysator eine Palladiumverbindung umfaßt, in Kombination mit einem Ligand, der dreiwertigen Phosphor umfaßt.

3. Verfahren nach Anspruch 2, worin der Katalysator Palladium(II)-acetat ist, in Kombination mit Triphenylphosphin.

4. Verfahren nach Anspruch 2, worin der Katalysator ein Bis(triphenylphosphin)dichlorpalladium(II)-Komplex ist.

5. Verfahren nach irgendeinem der Ansprüche 1 bis 4, worin die Reaktionstemperatur 120 °C bis 150 °C beträgt.

6. Verfahren nach irgendeinem der Ansprüche 1 bis 5, worin die Reaktionszeit 0,25 bis 10 Stunden ist.

7. Verfahren nach irgendeinem der Ansprüche 1 bis 6, worin die Reaktion in Gegenwart eines Lösungsmittels durchgeführt wird.

8. Verfahren nach Anspruch 7, worin da Lösungsmittel N-Methylpyrrolidon ist.

## Revendications

1. Procédé de production de nabumétone, qui consiste à faire réagir du 2-bromo-6-méthoxynaphtalène avec du 3-butène-2-ol en présence d'un catalyseur.

2. Procédé suivant la revendication 1, dans lequel le catalyseur comprend un composé de palladium en association avec un ligand contenant du phosphore trivalent.

3. Procédé suivant la revendication 2, dans lequel le catalyseur est formé d'acétate de palladium (II) en combinaison avec de la triphénylphosphine.

4. Procédé suivant la revendication 2, dans lequel le catalyseur est un complexe bis(triphénylphosphine)dichloropalladium (II).

5. Procédé suivant l'une quelconque des revendications 1 à 4, dans lequel la température de la réaction va de 120 à 150°C.

6. Procédé suivant l'une quelconque des revendications 1 à 5, dans lequel la durée de la réaction va de 0,25 à 10 heures.

7. Procédé suivant l'une quelconque des revendications 1 à 6, dans lequel la réaction est conduite en présence d'un solvant.

8. Procédé suivant la revendication 7, dans lequel le solvant est la N-méthylpyrrolidone.
